# EUROPEAN PATENT SPECIFICATION

(11) **EP 3 930 877 B1**
(45) Date of publication and mention of the grant of the patent: **02.08.2023**
(21) Application number: 20707441.0
(22) Date of filing: 28.02.2020
(51) Int. Cl.: A61M 1/16, B01D 61/58, B01D 63/02, B01D 61/14, B01D 61/44, B01D 61/00, B01D 61/42

(54) **PRODUCTION OF CONCENTRATED SPENT DIALYSATE**
HERSTELLUNG VON KONZENTRIERTEM VERBRAUCHTEM DIALYSAT
PRODUCTION DE DIALYSAT USAGÉ CONCENTRÉ

(30) Priority: 28.02.2019 DK PA201970140
(43) Date of publication of application: 05.01.2022
(73) Proprietor: Aquaporin A/S, 2800 Kongens Lyngby (DK)
(72) Inventor: ANDERSEN, Mads Friis, 2500 Valby (DK); MØLLER, Michael Holm, Singapore 648316 (SG); JØRGENSEN, Pernille Gramstrup Lund, 2800 Kgs. Lyngby (DK)
(74) Representative: Nordic Patent Service A/S
(86) International application number: PCT/EP2020/055351
(87) International publication number: WO 2020/174097

(56) References cited:
- EP-A1- 2 900 296
- WO-A2-2009/083011
- DE-A1- 19 540 079
- US-A1- 2014 158 623
- US-A1- 2017 065 762

## Description

### TECHNICAL FIELD

The disclosure relates to a method for producing a concentrated spent dialysate and to a hemodialysis treatment apparatus.

### BACKGROUND

In Denmark approximately 5000 people have end stage renal disease (ESRD), and about 2500 of these people get a kidney transplantation while the rest of the patients are depending on chronic dialysis treatment. Dialysis is a technical treatment that replaces the kidney's overall function to filtrate the blood and let the waste materials leave the body with the urine while retaining nutrients.

Generally, the patient is connected to a hemodialysis machine using catheters inserted into the patient's veins and arteries to connect the blood flow to and from the hemodialysis machine. As blood passes through a dialyzer in the hemodialysis machine, the dialyzer removes the waste, toxins and excess water from the patient's blood and returns the blood to infuse back into the patient. A large amount of dialysate, for example about 120 litres, is used to dialyze the blood during a single hemodialysis therapy. The spent dialysate is then discarded.

The mortality rate of the chronic dialysis patients is about 20% per year. The mortality is mainly due to cardiovascular disease. However, some patients have been known to live 30-40 years with chronic dialysis treatment. Better survival can be obtained through more intensive hemodialysis regimes than the current standard treatment, which consists of four hours of treatment three times a week. Hemodialysis sessions on about 6 hours 5-6 times a week have the potential to improve the survival rates.

These kinds of sessions are so frequent that they are undertaken by the patients themselves in their own homes. This way the patients do not have to go to the hospital every day. However, the home-based dialysis sessions are very expensive and space consuming, due to the installation of a stationary water purification system and a large dialysis machine. Furthermore, the quality of life for the patient is severely reduced as the patient's mobility is restricted.

The amount of water used for dialysis treatments are both massive and expensive. The ultra-pure water is produced from tap water which has to undergo a very intensive cleaning procedure to remove contaminants. A hemodialysis patient uses about 400 litre of ultra-pure water per week and up to 25,000 lifters per year.

The production of ultrapure water to produce a high quality and purity water is an essential step of hemodialysis to prevent unwanted organic and inorganic chemicals naturally found in, e.g., tap or bottled water entering the patient's body. Typically, a combination of different devices and processes is used for obtaining ultrapure water according to local availability and specifications of tap water. In some countries an iron remover is necessary to remove high iron concentration from the water. In Denmark, a water softener is necessary to remove the excess amount of calcium and magnesium from the water. A charcoal filter may be used to remove chloramine from the water. Typically, a reverse osmosis filter is used to remove more than 95% of the remaining ions and some bacteria and this is the primarily water purification process of choice for most application and the most expensive part of the process. A typical reverse osmosis plant has a recovery of around 40-55% ultrapure water and hence a lot of water is wasted during the RO process.

During hemodialysis, the ultrapure water is mixed with an acid concentrate and dissolved sodium carbonate, which provides the dialysate for the treatment. In a typical hemodialysis system, the sodium bicarbonate is dissolved with RO water and the acid concentrate is diluted with RO water before these two flows can be mixed together. After mixing, the mixed flow enters the dialyser where the substances diffuse with the patient's blood to remove waste compounds from the blood. The dialysate must have the same concentrations of salts and nutrients as the blood in order to be effective and not to deplete the patient's body of essential salts and nutrients.

In WO2010/146365 A1 it has been suggested to recover purified water lost during hemodialysis by the use of a liquid membrane system comprising vesicles having a bilayer into which bio channels such as aquaporin water channels have been incorporated and wherein the vesicles further comprise a stabilising oil phase. In order to accomplish this, it was suggested to create a salt gradient and a counter-current mimicking the normal kidney function across said liquid membrane, which will then constitute the necessary driving force for a forward osmosis processes. Talaat (Saudi J Kidney Dis. Transpl.,21(4):748-749, 2010) later proposed to use forward osmosis for dialysis fluid regeneration where theoretically up to 50 % of spent dialysis fluid water may be retrieved. However, due to limitations on draw solutes such as salts and glucose, Talaat concludes that a modified dialysis therapy prescription such as a prolonged daily therapy is necessary, and he further proposes to use a multilayer FO plate and frame module to be specifically built for testing the hypotheses.

It has been suggested in WO 2015124716 to recycle the wastewater from the spent dialysate. More specifically, it has been suggested to recycle a filtrate derived from a patient's blood or plasma during a renal replacement therapy process by deploying a flux of water from said spent fluid by forward osmosis. The method includes a forward osmosis (FO) unit comprising a forward osmosis membrane having aquaporin water channels. The membrane has a feed side and a draw side wherein the feed side is in fluid communication with the filtrate from a patient undergoing said hemofiltration, and the draw side is in fluid communication with a supply of an electrolyte concentrate.

WO 2014/158418 A1 suggests systems and methods for hemodialysis or peritoneal dialysis, that includes the initial treatment of a spent dialysate with a carbon source and an urease source before an electrodialysis or electrodeionization (ED/EDI) process is performed. The urease source is applied for facilitating the breakdown of urea into ammonium and ammonia, which may be cleared from the fluid in a subsequent cation exchanger. After the treatment the fluid is returned to the patient after optional adjustments.

Alternative approaches to obtaining ultrapure water include e.g.: US20170189597A1, which describes a portable dialysis machine to be used in alternative environments, e.g. in disaster relief settings or underdeveloped regions. The machine includes solar panels and an atmospheric water generator for extracting water from ambient air which then passes a FO unit for purification.

Other portable home dialysis systems exist, e.g. Nxstage System One^{®}, however this system requires the installation of water treatment systems or may be connected to a separate device, PureFlow SL, which utilises a replaceable cartridge including the necessary filters, deionisers, sorbents and so forth to generate dialysis solutions at home.

The object of the present invention is to obtain a high recovery rate of water either by recycling the water of the spent dialysate or by purifying tap water at a home setting to offer a portable dialysis treatment to dialysis patients or a least a mobile solution which the patients can drag around in their homes. The higher the water recovery rate the lower the weight that needs to be carried by the patient. As a standard treatment produces around 120 l spent dialysate a recovery rate of, e.g., 80% will minimize weight to 24 kg. A mobile solution would not only reduce the amount of wastewater but also offer the possibility of a gentler way of hemodialysis due to the possibility of prolonged treatment periods. The treatment using prolonged treatment periods is expected to be less stressful for the body as it resembles the normal function of the kidney, and thus has the potential of prolonging the patient's life as well as improving the patient's quality of life.

### SUMMARY

It is an object to provide a method for producing a concentrated spent dialysate comprising the steps of reducing the amount of electrolytes in a spent dialysate by electrodialysis and de-watering the spent dialysate by a forward osmosis operation. Accordingly, the invention provides a method for producing a concentrated spent dialysate according to claim 1 and a hemodialysis treatment apparatus according to claim 9.

The forward osmosis operation of the present invention usually comprises a membrane module comprising a membrane capable of allowing water to pass and rejecting certain solutes. The membrane module also comprises a compartment for the feed solution, which may be referred to as the feed side, and a compartment for a draw solution, which may be referred to as the draw side. In a certain embodiment of the present invention, the membrane comprises nano-porous water channels. The nano-porous water channels ideally have a size allowing only water to permeate. However, in reality also a minor amount of refluxed solutes penetrates the membrane from the spent dialysate to the concentrated dialysate. The presence of nano-porous water channels reduces or eliminates reabsorption of all or most other solutes from the spent dialysate. Moreover, it is preferred that the membrane module of the method comprises a membrane comprising aquaporin water channels, which, due to their highly selective nature, only pass pure water molecules. The forward osmosis operation comprising the forward osmosis membrane having nanopores such as aquaporin water channels may be provided in the form of a membrane module as disclosed in WO14108827 A1, WO2017137361 A1, WO2018/141985, or WO18087289.

In a certain embodiment, the membrane comprises an active layer comprising immobilized aquaporin water channels, and a support layer. The active layer may be a cross linked aromatic amide layer, preferably formed by interfacial polymerization, wherein stabilized aquaporins are incorporated. The stabilized aquaporins may be in the form of vesicles being formed by self-assembly of amphiphilic matrix forming compounds in the presence of an aquaporin protein preparation as disclosed in WO2018/141985 or WO18087289. Alternatively, the aquaporin water channels may be stabilized by PEI (polyethyleneimine) as disclosed in WO2017137361 A1. Membranes prepared in this way have proven to be robust, to have high water transport capacity and low reverse flux of ions and low molecular weight substances.

The spent dialysate used in the present invention emanates from hemofiltration or hemodialysis apparatuses manufactured and sold by a number of companies including Omniflo (subsidiary of Transvivo Inc. Napa, CA, USA), Fresenius Medical Care (fmc-ag.com), Gambro (gambro.com) (now part of Baxter), Nipro (nipro.com), nxStage (nxstage.com) and Bellco (bellco.net).

In hemodialysis, the dialyzer functions partly as an artificial kidney. The dialyzer is typical in the form of a hollow fibre module with a bundle of hollow fibres (such as up to 20,000 fibres). The lumen of the hollow fibres forms the "blood compartment" of the dialyzer and the space surrounding the fibres forms the "dialysate compartment".

In peritoneal dialysis, the peritoneum in connection with the infused dialysate functions partly as an artificial kidney and the patient's peritoneal membrane functions as a semipermeable membrane across which fluids and dissolved substances are exchanged from the blood. Fresh hyperosmotic dialysate solution is introduced through a permanent tube into the abdominal cavity and spent dialysate is flushed out either every night while the patient sleeps (automatic peritoneal dialysis) or via regular exchanges throughout the day (continuous ambulatory peritoneal dialysis).

The actual composition of spent dialysate solution varies from patient to patient and from time to time. Metabolic changes appear in patients suffering from kidney failure resulting in elevated urea levels in the patient's blood plasma along with elevated levels of toxic metabolites such as indoles, phenols, hormones, oxalate and protein degradation products.

A number of technologies for reducing the amount of electrolytes exist, including electrodialysis, ion exchange, microfiltration, and nanofiltration. According to the present invention electrodialysis is used as a pre-treatment of the spent dialysate before treatment in a forward osmosis process.

Suitable electrodialysis (ED) methods include electrodeionization (EDI), and electrodialysis reversal (EDR). In general, electrodialysis is used to remove ions from the spent dialysate under the influence of an applied electric potential difference. The electrolysis process may be performed in a configuration called an electrodialysis cell that includes a feed (diluate) compartment and two concentrate (brine) compartments. The diluate compartment has an anion selective membrane positioned towards the anode and a cation selective membrane placed towards the cathode. Multiple electrodialysis cells may be arranged into a configuration called an electrodialysis stack, with alternating anion and cation exchange membranes forming the multiple electrodialysis cells. By the electrodialysis process dissolved species are moved away from the feed stream, i.e. the spent dialysate.

Electrodeionization (EDI) is a technology that combines ion exchange resins and ion-selective membranes with direct current to remove ions from the spent dialysate. The spent dialysate passes through one or more chambers filled with ion exchange resins held between cation or anion selective membranes. Ions that become bound to the ion exchange resins migrate to a separate chamber under the influence of an externally applied electric field. This also produces the H+ and OH- ions necessary to maintain the resins in their regenerated state. Ions in the separate chamber may be discarded. EDI modules are obtainable as DOW OMEXELL modules, Zapwater EDI modules from Snowpure, and Ionpure^{®} modules from SIEMENS.

Electrodialysis reversal (EDR) includes that electric current transfers dissolved salt ions through an electrodialysis (ED) stack. The stack has alternating layers of anionic and cationic ion exchange membranes that work together to separate dissolved salts from the water. To avoid solutes to deposit on the membrane, the polarity of the applied electrical potential is reversed in certain time intervals, resulting in the removal of charged solutes that have precipitated on the membranes. The polarity of the electrodes may be changed every 30 min to 2 hours. As an example the NEXED module obtainable from Evoqua Water Technology may be used.

The electrodialysis may not fully deplete the spent dialysis fluid for ions because the level of electrolyte removal cannot be allowed to go below a certain limit. Below a certain limit water splitting occurs and a considerable amount of energy is wasted in splitting the water. Furthermore, water splitting will result in the production of oxygen at the anode and hydrogen at the cathode. Therefore, for various reasons water splitting it is not desired.

In the pre-treatment step the amount of electrolytes in the spent dialysate is reduced by at least 20% by the electrodialysis. Suitably, the amount of electrolytes is reduced by at least 30%, such as at least 40%. In another implementation of the invention, the amount of electrolytes in the spent dialysate is not reduced by more than 80%.

While electrodialysis are useful for removing charged solutes of the spent dialysate, the uncharged or neutral solutes remain in the spent dialysate. However, the uncharged or neutral solutes are at least partly removed in the subsequent forward osmosis process.

While a number of different module designs, including spiral wound membrane module, hollow fibre module, plate-and-frame modules etc. may be used for de-watering the spent dialysate, it is generally preferred to use one or more hollow fibre module(s) in which the de-watering by a forward osmosis operation of the spent dialysate is performed by passing the spent dialysate through the feed side of a first hollow fibre module, whereas a draw solution is passed through the draw side of the first hollow fibre module. In an embodiment of the invention the feed side is the lumen of the fibres and the draw side is the shell side of the hollow fibre module. In another embodiment of the invention the feed side is the shell side of the hollow fibre module and the draw side is the lumen of the fibres of the hollow fibre module. Generally, the rejection of the solutes is regarded to be higher when the feed is treated in the lumen of the fibres, whereas the water flux is regraded to be higher when the feed is treated on the shell side of the hollow fibre module. The selective layer may be present on either the inner (lumen) side of the hollow fibres or on the outside of the hollow fibres. In a presently preferred embodiment of the invention the selective layer is present in the inner (lumen) side of the hollow fibres, the feed is treated in the lumen of the hollow fibres, and the concentrated dialysate is directed to the shell side of the hollow fibre module.

The hollow fibre module has the advantage over the spiral wound module and the plate-and-frame module of being compact and furthermore has a well-known form factor for use in hemofiltration apparatuses. In addition, the module can be easy to replace after use in single use applications in continuous renal replacement therapies.

According to the invention the draw solution is concentrated dialysate. A variety of concentrate formulations for preparing dialysate solutions for hemodialysis or peritoneal dialysis are well known in the art. These formulations vary not only with respect to specific constituents but also with respect to the concentration of these constituents. Generally, concentrate formulations include sodium chloride as the major constituent and potassium chloride, calcium chloride and magnesium chloride as minor substituents. Also, dextrose (glucose) may be included.

Sodium acetate and/or sodium bicarbonate are also included as a buffer source to correct for metabolic acidosis. With acetate buffer, all of the constituents can be combined into a single concentrate. With bicarbonate buffer, two concentrates are necessary to prevent the precipitation of calcium and magnesium as carbonate salts.

Conventional two-part bicarbonate-based dialysis solutions are prepared by mixing an "acid" concentrate, a "base" concentrate and water. Normally, the acid concentrate includes all of the acid, dextrose, calcium, magnesium, potassium and some portion of the physiologic requirement for sodium chloride whereas the base concentrate includes sodium bicarbonate and the balance of the required sodium chloride. In some commercial formulations of dialysate concentrates, the sodium chloride content of the base concentrate is zero. Since acetic acid is a liquid at room temperature, most of the acid concentrates using acetic acid are liquid products, whereas the base concentrates are produced both as powder and liquid concentrates. Many other combinations of acid and base concentrates that are commercially available are specific to the dialysis solution preparation methods and delivery equipment.

Specific examples of dialysate concentrate systems are (in brackets are mentioned the dilution ratios as volume of acid concentrate to volume of base concentrate to volume of water) the Renasol^{®} bicarbonate hemodialysis concentrate series (1:1.83:34) and the Centrisol^{®} hemodialysis concentrate series (1:1.72:42.28) from Medivators (www-medivators.com) (formerly Minntech), the NaturaLyte^{®} Liquid Acid Concentrates series (1:1.72:42.28) and the Citrasate^{®} Citric Acid Concentrate series (1:1.72:42.28) from Fresenius Medical Care, the RenalPure^{®} Liquid Acid Concentrate/ SteriLyte^{®} series (1:1.83:34, 1:1.72:42.28 and 1:1.225:32.775) from Rockwell Medical (www-rockwellmed.com) and the BiCart Select^{®} Citrate series (1:2:197) from Gambro (www.gambro.com). In the examples reported herein SoftPac^{™} C298 is used as the acid dialysate concentrate for bicarbonate-based hemodialysis. 3.5L of the SoftPac^{™} concentrate was diluted 1+44 (1:45) dilution for the production of 157L dialysate when mixed. Electrolyte concentration after dilution together with BiCart^{®} cartridge and machine setting Na⁺/HCO₃⁻ = 140/34mmol/L.

The diluted concentrated dialysate is delivered to a hemodialysis treatment apparatus, optionally after adjustment of the concentration of the components. The effect of using the optionally adjusted diluted dialysate in the dialysis machine is that the water withdrawn from the spent dialysate is used directly as part of the fresh dialysate fed to the hemodialysis treatment apparatus. In other words, an amount of water can be recycled from the spent dialysate to the fresh dialysate.

In a certain embodiment of the invention, the first hollow fibre module is connected with second or further hollow fiber module(s), such that the partly de-watered spent dialysate solution exiting the feed side of the first hollow fiber module is further de-watered in the feed side of the second or further hollow fiber modules. The experiments reported herein surprisingly shows that the treatment of the spent dialysate in two or more consecutively arranged hollow fiber modules increases the recovery of water from the spent dialysate beyond what could be expected by the increase in membrane area. In a certain aspect of the invention, three hollow fiber modules are consecutively arranged, such that the spent dialysate is partly de-watered in feed side of a first hollow fiber module. The partly de-watered spent dialysate is the subsequently treated in the feed side of a second hollow fiber module to further withdraw water. The partly dewatered spent dialysate leaving the feed side of the second module is finally treated in the feed side of the third hollow fiber module to produce the concentrated spent dialysate. A fourth or further hollow fiber module may be present in the sequence for treating the partly dewatered dialysate. Between the hollow fiber membrane modules, it may be needed or necessary to provide the partly dewatered dialysate with a further pressure, e.g. by adding a pump in the passageway between the modules. A suitable pump is a peristaltic pump, however other pumps of the positive displacement type may also be used.

The draw solution being the concentrated dialysate may be delivered to the two or further modules in parallel or in series. When the concentrated dialysate is added to the membrane modules in parallel, the same concentration of the concentrated dialysate is added the inlet of each membrane module. In general, a manifold connected to a common reservoir may distribute the concentrated dialysate to the inlets of the modules. At the outlet of each of the modules the diluted dialysate is collected and pooled for further adjustment before it is used as a dialysate in a dialyser.

In the event the concentrated dialysate is delivered to membrane modules in series it may be added to the sequentially arranged modules co-current or counter-current.

When the concentrated dialysate is delivered to the sequence of membrane modules co-currently, the concentrated dialysate is added to the inlet of the first modules for treating the spent dialysate. At the outlet of the first module, the partly diluted dialysate is conveyed to the second or further module for treating the partly dewatered spent dialysate. At the outlet of the second or further module the diluted dialysate is collected and adjusted with sodium bicarbonate and water if needed, before being used as dialysate.

When the concentrated dialysate is delivered to the sequence of membrane modules counter-currently, the concentrated dialysate prior to dilution in the first hollow fibre module has been diluted in second or further hollow fibre module(s). In other words, the concentrated dialysate is first delivered to the inlet of the last module treating the partly dewatered spent dialysate. The partly diluted concentrated dialysate exists at the outlet of the last module and is entered into the inlet of the penultimate membrane module. It is generally preferred to deliver the concentrated dialysate and the spent dialysate counter-currently because the concentration difference resulting in a more effective dewatering of the spent dialysate. However, in some applications it may be desired with less electrolytic stress due to different electrolyte concentrations across the membrane and then the co-current treatment of the fluids can be applied.

The membrane area may be essentially identical or different for each of the membrane modules. It may be convenient to use the same type of module for each of the modules in the series because it reduces the complexity of the system. Generally, however, the membrane area of the first membrane module is larger than the membrane area of the second or further membrane module. In the first module, water is withdrawn from the spent dialysate. Consequently, the membrane area can be reduced in the subsequent membrane module because the volumetric stream is lower. In a preferred aspect of the invention, the first forward osmosis module has a membrane area twice as large as the second or further membrane module(s). More preferred, the first membrane module has a membrane area 2.5 times, 3 times or more higher than the second membrane module. If a third module is present in the sequence of modules, the area of the second membrane module is also higher than the third module such as 50%, 75%, or 100% higher than the membrane area of the third module. Smaller modules downstream of the spent dialysate have the advantage of a more effective utilization of the available membrane area. As the membrane area usually is the expensive part of a membrane module, the expenses for the present method may be reduced considerably be reducing the membrane area in the second or subsequent membrane module.

As an alternative to using membrane modules with decreasing membrane area, different numbers of modules of essentially the same size can be used at each stage. Thus, in certain embodiment the first membrane stage may comprise 5 to 10 forward osmosis modules operating in parallel, the second membrane stage can comprise 2 to 5 forward osmosis modules operating in parallel, and a third stage can comprise 1 to 2 forward osmosis modules operating in parallel.

In principle, the spent dialysate may have any temperature as long as it remains a liquid. It may be suitable for the present method to treat the spent dialysate at a temperature above room temperature, such as 5°C or more above temperature. Thus, in a preferred aspect the spent dialysate is treated by forward osmosis at a temperature of 30°C or above. Suitably, the spent dialysate is treated at a temperature at or slightly below body temperature, i.e. at a temperature between 33 to 38°C. It has surprisingly been shown by experiments that the water flux increases with increased temperature, thereby increasing the water recovery.

The pressure difference across the membrane also referred to as the transmembrane pressure (TMP) may be increase if the forward osmosis membrane is robust enough to withstand the pressure. Thus, in a preferred embodiment of the invention, the transmembrane pressure is 1 bar or above. In a preferred embodiment of the invention, the transmembrane pressure is at least 2 bar or more, such as 3 bar or more. The pressure may suitably be provided by a pump positioned in front of the first module.

A salt may be added to the draw solution, i.e. the concentrated dialysate or the partly diluted dialysate between membrane modules to increase the salt concentration. In a preferred aspect, sodium bicarbonate solution is added to the partly diluted dialysate between the first hollow fibre module and the second or the further hollow fibre module(s), or between the second hollow fibre module and the further hollow fibre module(s). When deciding the site of adding the sodium bicarbonate solution due consideration has to be given to the concentration of certain ions to avoid precipitation conditions. As discussed above, sodium carbonate or other basic compounds are normally added to the dialysate prior to the use in the dialyzer. The present inventors have realized that by adding the sodium carbonate to the draw solution between the modules the water flux increases.

The present invention is not confined to a particular type of forward osmosis membrane, as long as the membrane has the ability of allowing water to pass while retaining salt and other solutes. In a particularly preferred embodiment of the invention the membrane of the forward osmosis unit comprises aquaporin water channels. The presence of aquaporin water channels greatly increases the flux thereby allowing a smaller membrane area to be used for filtering a certain amount of spent dialysate.

The present invention also relates to a hemodialysis treatment apparatus comprising:
a) an ultrafiltration unit allowing for exchange of solutes of a patient's blood plasma and a dialysate, resulting in a stream of cleaned blood for returning to the patient and a stream of spent dialysate;
b) an electrodialysis device capable of reducing the amount of electrolytes in the spent dialysate,
c) a forward osmosis unit comprising a membrane having a feed side and a draw side, the membrane being substantially impervious to solutes and essentially allowing only water to permeate, wherein a stream of spent dialysate from the ultrafiltration unit, is in fluid communication with the feed side and a stream of concentrated dialysate is in fluid communication with the draw side, resulting in a stream of dialysate, optionally after adjustment of the concentration of the solutes, for use in the ultrafiltration unit and a stream of concentrated spent dialysate; and
d) a pump for pumping blood plasma from the patient to the ultrafiltration unit.

The recovered water of the spent dialysate solution is used in the preparation of a fresh dialysate solution or fresh replacement or reconstitution fluid for hemofiltration and kidney disease dialysis. In other words, the forward osmosis treatment results in a flux of water from the spent dialysate fluid into a supply of electrolyte replacement concentrate. Thus, the hemodialysis treatment apparatus of the invention offers the possibility of recirculating a part of the water in the spent dialysate to the dialysate used in the dialyzer. As a result, a spent dialysate concentrate is produced, which may be discarded or treated further in a subsequent process.

In a variant of the apparatus, a water source different from spent dialysate is conveyed to the forward osmosis unit for diluting the concentrated dialysate. As an example, the water source is tap water and the spent dialysate is discarded or treated by other means.

In a preferred aspect of the invention, the forward osmosis unit is a hollow fibre module.

In an embodiment of the invention the feed side of the first forward osmosis unit is connected to the feed side of a second or further forward osmosis units, such that the partly de-watered spent dialysate solution exiting the first forward osmosis unit is further de-watered in the feed side of the second or further hollow fibre modules.

In an implementation of the invention, the first forward osmosis unit has a membrane area twice as large as the second or further membrane module(s).

In an embodiment, the apparatus comprises a heater capable of heating the spent dialysate prior to treatment in the forward osmosis unit.

In another embodiment, the apparatus comprises a pump configured for providing a transmembrane pressure in the forward osmosis unit of 1 bar or more.

In a further embodiment, the conduit between the first forward osmosis and the second or further osmosis unit(s) is supplemented with a further conduit, which is connected to a source of sodium bicarbonate.

In a further embodiment of the invention, the apparatus comprises a pump for pumping the concentrated dialysate to the forward osmosis unit, or a pump for suction of the diluted dialysate from the forward osmosis unit.

In a further embodiment, the apparatus comprises a mixing device for adjusting the dialysate from the forward osmosis unit with a sodium carbonate solution. Suitably, the apparatus comprises a conductivity sensor after the mixing device for feed-back regulation of the adjustment of the dialysate from the forward osmosis unit with the sodium carbonate solution.

Another non-claimed aspect of the present disclosure relates to a method for producing a dialysate for use in a hemodialysis treatment comprising the steps of:
Providing a forward osmosis unit comprising a membrane having a feed side and a draw side, the membrane being substantially impervious to solutes and essentially allowing only water to penetrate,
Allowing a stream of a water source to be in fluid communication with the feed side and a stream of concentrated dialysate to be in fluid communication with the draw side, to produce a stream of diluted dialysate and a stream of de-watered water source,
Wherein the water source is tap water.

Tap water is easy available allowing the patient to receive treatment in their homes and elsewhere, thus improving the life quality of the hemodialysis patient. Furthermore, the expenses for producing reverse osmosis water can be saved. Additionally, the mortality rate can be expected to drop as the patient now can undergo more frequent and longer hemodialysis treatments producing less stress on the body.

Tap water is a general term for water produced centrally at a water plant and then delivered to a plurality of induvial households or other users through a piping system. The quality of the tap water varies in dependence of the geographical location and the water source used by the water plant.

In an implementation of the second aspect a sodium bicarbonate solution is added the diluted dialysate before it is used in a hemodialysis treatment.

In another implementation of the second aspect of the invention the diluted dialysate is adjusted with the addition of water or solutes to obtain a conductivity of the diluted dialysate between 12-16 mS/cm before usage in a hemodialysis treatment.

In another implementation of the second aspect the tap water contains an amount of solutes of 3000 ppm or less, such as 2000 ppm or less, and suitably 1500 ppm or less.

In another implementation of the second aspect the tap water contains an amount of solutes of 30 ppm or higher, such as 50 ppm or higher, and suitably 100 ppm or higher.

In another implementation of the second aspect the membrane of the forward osmosis unit comprises aquaporin water channels.

In a third non-claimed aspect of the disclosure there is provided a hemodialysis treatment apparatus comprising
a forward osmosis unit comprising a membrane having a feed side and a draw side, the membrane being substantially impervious to solutes and essentially allowing only water to penetrate, said feed side of the forward osmosis unit being configured for receiving a stream of a water source and said draw side being configured for receiving a stream of concentrated dialysate, thereby producing a stream of diluted dialysate and a stream of concentrated water source;
an ultrafiltration unit configured to receiving a stream of a patient's blood or plasma and a stream of dialysate, allowing for exchange of solutes of the patient's blood or plasma and the dialysate, thereby producing a stream of cleaned blood for returning to the patient and a stream of spent dialysate,
a pump for pumping blood or plasma from the patient to the ultrafiltration unit,
wherein the forward osmosis unit is configured for receiving tap water as the water source.

In an implementation of the third aspect the ultrafiltration unit is configured for delivering the stream of spent dialysate to a drain.

In an implementation of the third aspect the apparatus further comprises a mixing device configured for receiving the diluted dialysate and a sodium carbonate solution for production of a dialysate for use in a dialysis treatment.

In an implementation of the third aspect the mixing device further is configured for receiving a pH adjusting solution.

The foregoing and other objects are achieved by the features of the independent claims. Further implementation forms are apparent from the dependent claims, the description and the figures. These and other aspects will be apparent from and the embodiment(s) described below.

### BRIEF DESCRIPTION OF THE DRAWINGS

In the following detailed portion of the present disclosure, the aspects, embodiments and implementations will be explained in more detail with reference to the example embodiments shown in the drawings, in which:
Fig. 1 is a schematic representation of the recirculation of water from the spent dialysate to the dialysate.
Fig. 2 illustrate the experimental setup.
Fig. 3 shows an embodiment of the forward osmosis operation using two modules in series.
Fig. 4 shows an embodiment of the forward osmosis operation using three modules in series.
Fig. 5 discloses the provision of the pump at the feed side for providing a transmembrane pressure of the embodiment shown in Fig. 4.
Fig. 6 discloses the supplementing of the partly diluted draw solution with a sodium bicarbonate solution between the first and the second module of the embodiment shown in Fig. 5
Fig. 7 shows the provision of a pre-treatment of the feed solution to reduce the solute concentration prior to being fed into the first module.

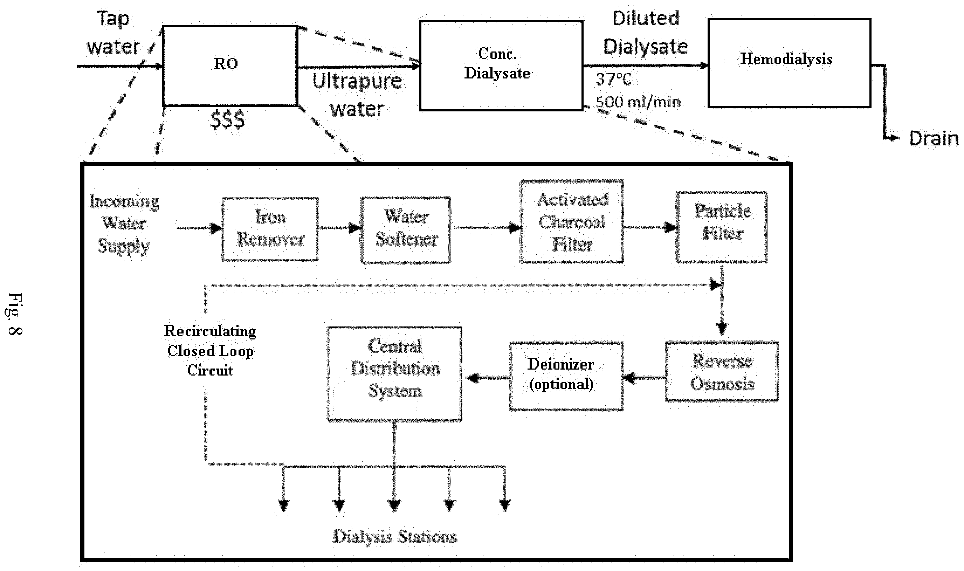

Fig. 8 shows a flow chart of currently used RO process.

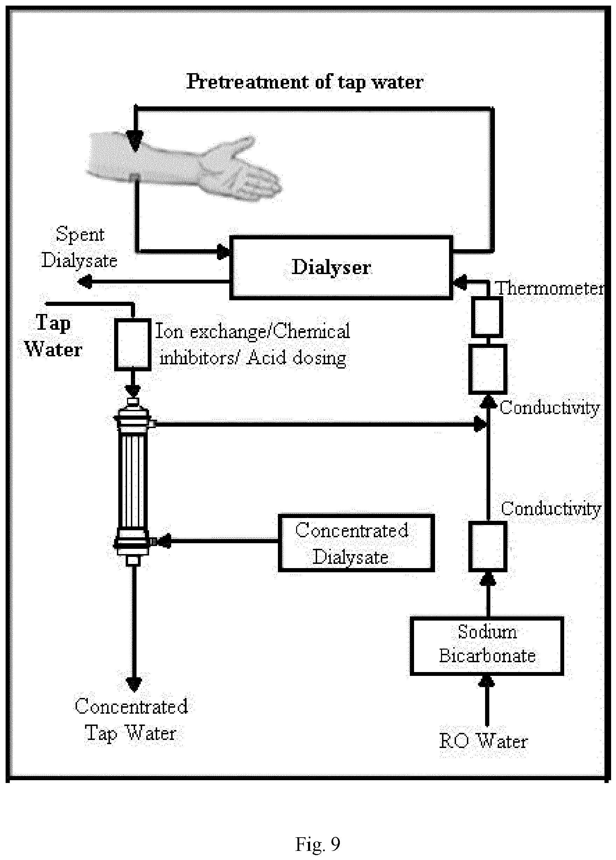

Fig. 9 discloses a schematic representation of the use of tap water to produce the dialysate.
Fig. 10 shows a general setup for the use tap water in the production of dialysate.
Fig. 11 shows a specific setup for the use tap water in the production of dialysate.

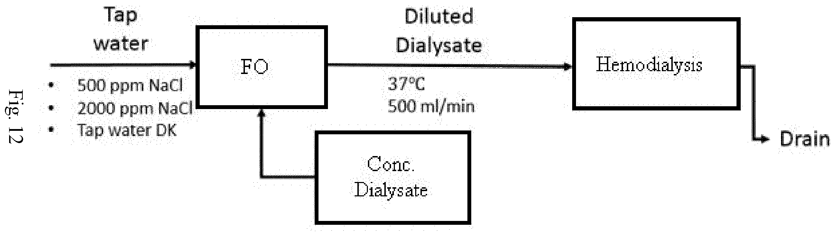

Fig. 12 shows a flow chart of the experiment reported in the examples.
Fig. 13 shows the experimental results obtained at 60% recovery.
Fig. 14 shows the feed flow over time for obtaining 60% recovery.
Fig. 15 shows the permeation and dilution factor over time for obtaining 60% recovery.
Fig. 16 shows the recovery over time for obtaining 60% recovery.
Fig. 17 shows the conductivity over time for obtaining 60% recovery.
Fig. 18 shows the temperature over time for obtaining 60% recovery.
Fig. 19 shows the conductivity at various dilutions for obtaining 60% recovery.
Fig. 20 shows the feed flow versus the recovery.

### DETAILED DESCRIPTION

The overall purpose of this invention is to explore the possibility for reducing the amount of water consumption during hemodialysis treatment. Besides saving water being a desired purpose, the reduced usage of water also makes it possible to produce a mobile dialysis treatment apparatus, which will create a better life for the patients.

The experiments presented herein have shown that it is possible to obtain a reduction of water usage of at least 50% or more, such as at least 60% or more, suitably at least 70% or more, preferably at least 80% or more, and more preferably at least 90% or more. Initial experiments have been conducted on a sodium chloride solution designed to have the same osmotic pressure as real spent dialysate. Subsequent experiments have been performed at a hospital (Rigshospitalet in Denmark) using real spent dialysate. In both instances the water recovery rate was examined. The experiments were conducted on different patients, on patients with different days of rest between treatments and on spent dialysate with different concentrations of waste compounds. Surprisingly, the results obtained based on spent dialysate from patients correlated well with the results obtained for the sodium chloride solution, i.e. the water recovery rates were essentially the same for spent dialysate from patients and the sodium chloride solution.

By building a setup with three different sized modules, addition of 3 bar transmembrane pressure (TMP) and the addition of sodium bicarbonate during the process, a water recovery of 74% was reached on the sodium chloride solution whereas a recovery rate of 76% was reached at for spent dialysate from patients. A simulated pre-treated spent dialysate experiment reached a recovery on 95%. A water recovery rate above 90% is generally regarded as desirable for a portable solution.

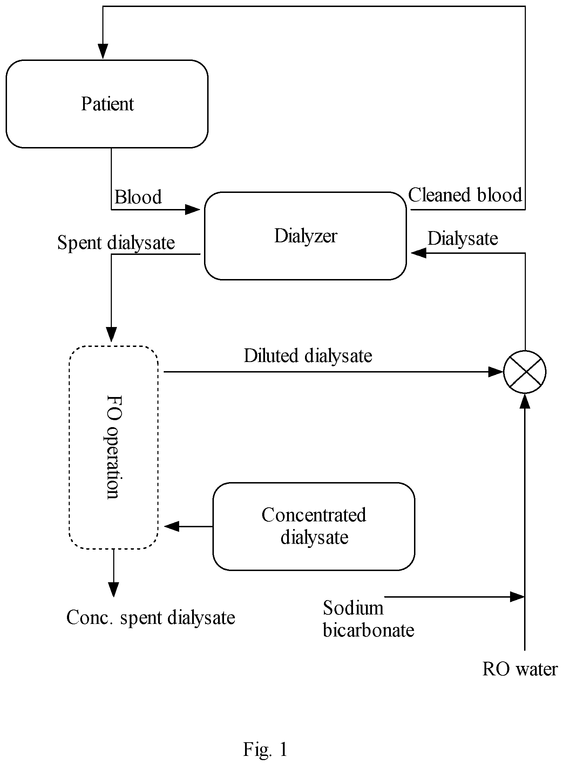

A mobile or portable hemodialysis treatment apparatus is devised by the present invention and the principle is shown in Fig. 1. The portable or mobile system comprises a dialyzer, a forward osmosis unit, and one or more pumps for circulating the fluids. Unless the elements of the portable or mobile system are fully integrated, the system also comprises tubes or hoses for connecting the elements. Notably, the patient's blood is connected through a tube or hose to the inlet of the dialyzer, i.e. the inlet of the ultrafiltration unit. After being dialyzed, the cleaned blood leaves the outlet of the dialyzer and is transported through a tube for delivery to the patent. The dialysate enters the inlet of the dialyzer and exchanges with the blood and the spent dialysate exits the dialyzer at the outlet as spent dialysate. The spent dialysate is transported through a tube from the outlet of the dialyzer to the inlet of a feed compartment of a forward osmosis module in a forward osmosis operation, optionally assisted by a pump. At the outlet of forward osmosis operation, the concentrated spent dialysate exits for further treatment or discharge. An aqueous solution of concentrated dialysate is delivered to the inlet of the draw compartment of the forward osmosis unit. In the forward osmosis unit water is transported over the membrane, thereby concentrating the spent dialysate and diluting the concentrated dialysate. The diluted dialysate exits the forward osmosis operation at an outlet and may be fed directly to the inlet of the dialyzer. However, usually, the diluted dialysate needs to be adjusted by addition of a sodium bicarbonate solution before entering the dialyzer.

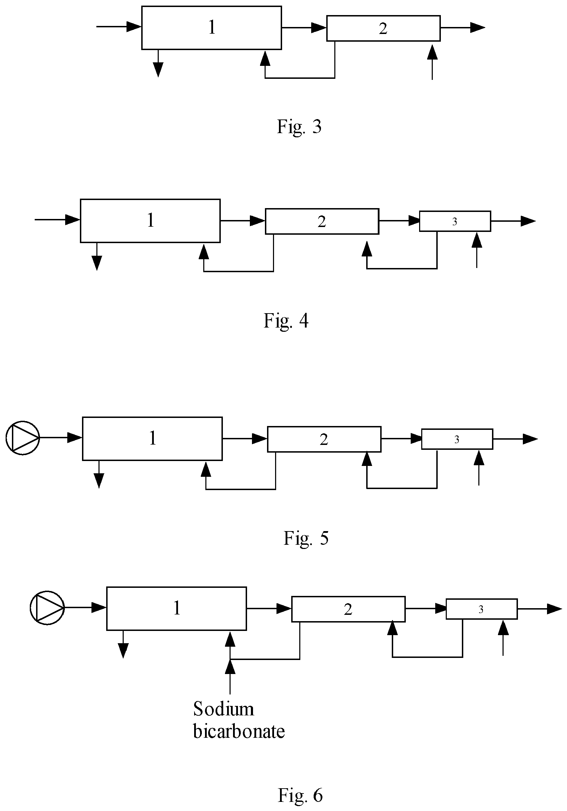

Various types of forward osmosis operations are disclosed in Figures 3 to 7. In figure 3, the forward osmosis operation comprises two forward osmosis modules arranged in series, i.e. the spent dialysate enters at the left arrow into the feed compartment of a first module. After partly dewatering the partly concentrated spent dialysate is transported to the feed compartment of a second module. In a preferred embodiment. Hollow fibre modules are used in the forward osmosis operation. While hollow fibre modules of the same size may be used, it is not necessary that the second module is of a similar size as a part of the water has been removed. The arrow at the right-handed side of the figure illustrates the introduction of a concentrated dialysate to the shell side of the second hollow fibre module. After the concentrated dialysate has been diluted in the second hollow fibre module, the partly diluted concentrated dialysate leaves at an outlet. The partly diluted concentrated dialysate is transported to the inlet of the first module for further dilution and the diluted dialysate exits at an outlet in the other end of the module.

Figure 4 shows a further development of the embodiment shown in figure 3. In the further development, a third forward osmosis module has been provided in series, such that the partly concentrated spent dialysate leaving the second module is transported to the feed side inlet of the second module. After being treated in the third forward osmosis module, the concentrated spent dialysate is discharged. The draw solution, i.e. the concentrated dialysate, enters at the inlet of the third module at the arrow on the right-handed side of the figure. The partly diluted dialysate is, after treatment in the third forward osmosis module, transported to the inlet of the second module and treated as indicated above.

Figure 5 shows a further development of the embodiment of figure 4, in which a pump is supplying the spent dialysate with a hydrostatic pressure before the spent dialysate enters the inlet of the first module.

Figure 6 illustrates a further development of the embodiment of figure 5, in which a solution of sodium bicarbonate and optional further salts are added to the partly diluted dialysate before entering the inlet of the first module.

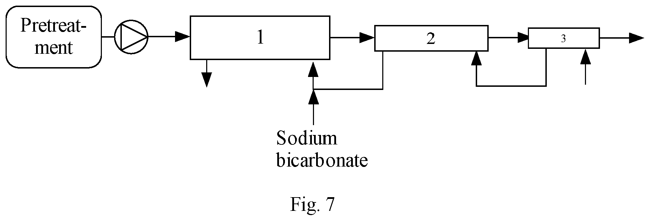

Figure 7 illustrates a further development of the embodiment of figure 6, in which the spent dialysate prior to being pumped into the feed compartment of the first osmosis unit is subjected to a pre-treatment to remove a part of the electrolytes. The pre-treatment may be conducted in an electrodialysis device.

### EXAMPLES

### Example 1 (comparative)

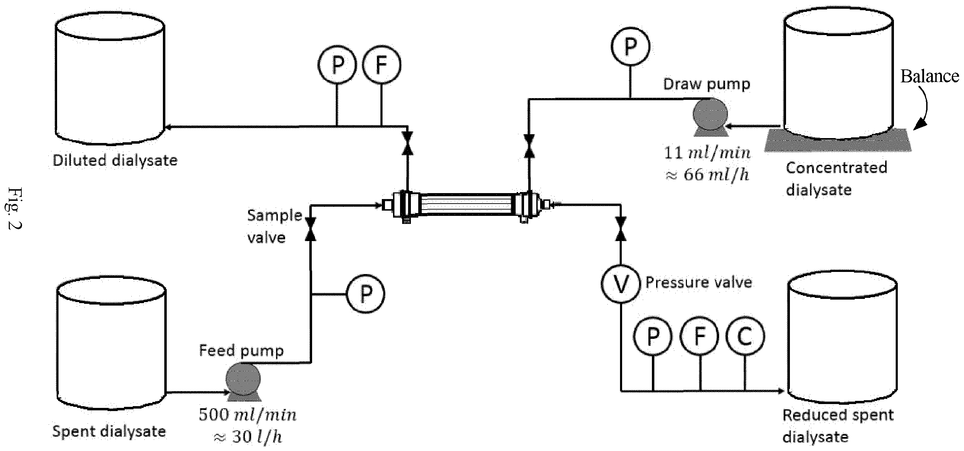

An experimental test set up was prepared as indicated in WO 2015/124716. A HFFO2 forward osmosis module (membrane area: 2.3 m²) available from Aquaporin A/S was used to concentrate spent dialysate by pumping the solution at a velocity of 500 ml/min to the lumen side of the module as illustrated on figure 2. The model spent dialysate was prepared by dissolving 228 g sodium chloride in 25 l water corresponding to an osmolarity of 0.27 osmol/kg. Water was drawn by forward osmosis through the membrane by a concentrated electrolyte solution entering the shell side of the module at a velocity of 11 ml/min. The concentrated electrolyte solution was diluted by the water drawn through the membrane and exits as diluted electrolyte solution. The spent dialysate is concentrated by the water being drawn through the membrane and leaves the other side of the fibre of the module as a concentrated dialysate.

The recovery rate is the percentage of water recovered from the spent dialysate. It is calculated as the permeation or volumetric flux of water drawn though the membrane (ml/min) divided by the volumetric flow of feed (ml/min), i.e. spent dialysate. For practical reasons, the data for calculating the recovery rate was gathered after a certain amount of time has elapsed or a certain amount of spent dialysate was treated in the module.

At a temperature of 18°C the recovery rate for the HFFO2 membrane module was calculated as 29.5% as an average of 2 runs.

By increasing the temperature to 37°C the recovery rate increased to 36% as an average of 2 runs.

### Example 2

As illustrated in Fig. 3 two modules were used in series as the FO operation in a setup otherwise similar to example 1. Thus, the HFFO2 module and a HFFO.6 module (membrane area 0.6 m²) available from Aquaporin A/S were positioned in series so that the spent dialysate first enters the lumen side of the HFFO2 module and then directly is fed to the lumen side of the HFFO.6 module.

The concentrated electrolyte solution, i.e. draw solution, was delivered to the shell side of the modules in counter-current with the feed solution. Thus, the concentrated electrolyte solution was first entered into the shell side of the HFFO.6 module for partial dilution and then into the shell side of the HFFO2 module to obtain the diluted electrolyte solution.

As the average of two runs, the recovery rate was calculated as 32%. The experiments were run at a temperature of 18.5°C.

### Example 3

As illustrated in fig. 4 three hollow fibre modules were used in series in a set up otherwise similar to example 1. Thus, a HFFO2 module, a HFFO.6 module, and a HFFO.3 module (membrane area: 0.6 m²) available from Aquaporin A/S were positioned in series so that the spent dialysate first enters the lumen side of the HFFO2 module, then directly is fed to the lumen side of the HFFO.6 module, and finally conveyed to the lumen side of the HFFO.3 module.

The concentrated electrolyte solution, i.e. draw solution, was delivered to the shell side of the modules in counter-current with the feed solution. Thus, the concentrated electrolyte solution was first entered into the shell side of the HFFO.3 module for partial dilution, and then into the shell side of the HFFO.6 module for further dilution, and then finally into the HFFO2 module to obtain the diluted electrolyte solution.

As the average of two runs, the recovery rate was calculated as 37%. The experiments were run at a temperature of 18.5°C.

As the average of two runs, the recovery rate was calculated as 37%. The experiments were run at a temperature of 18.5°C.

By increasing the temperature to 37°C the recovery rate increased to 43% as an average of 2 runs.

The model spent dialysate solution was exchanged with spent dialysate from real patients. At a temperature of 34°C spent dialysate from 3 patients were tested. In average the recovery rate was 45%, i.e. in agreement with the results obtained for the model spent dialysate solution.

### Example 4

The set up using 3 modules in series applied in example 3, was provided with a transmembrane pressure (TMP) to investigate the possible effect on the recovery rate. The system is illustrated in figure 5.

Based on 2 runs, a recovery rate of 51% was obtained when a transmembrane pressure of 1 bar was applied at a temperature of 37°C. When the transmembrane pressure was increased to 3 bar the recovery rate increased to 66%.

The model spent dialysate solution was exchanged with spent dialysate from real patients. At a temperature of 34 degrees Celsius spent dialysate from 3 patients were tested, applying a transmembrane pressure of 3 bar. In average the recovery rate was 66%, i.e., in agreement with the results obtained for the model spent dialysate solution.

### Example 5

The setup used in examples 3 and 4 was further changed by supplementing the system with addition of 15 ml/min sodium bicarbonate as shown in Fig. 6. Thus, the tube between the HFFO.6 and the HFFO2 module was interrupted by a valve allowing for the addition of the sodium bicarbonate solution.

The experiments showed a recovery rate of 74% as the average of 2 runs conducted at a temperature of 37°C and a TMP of 3 bar.

The model spent dialysate solution was exchanged with spent dialysate from real patients. At a temperature of 34 degrees Celsius spent dialysate from 3 patients were tested, applying a transmembrane pressure of 3 bar and addition of sodium bicarbonate (15mg/min). In average the recovery rate was 75%, i.e. in agreement with the results obtained for the model spent dialysate solution.

### Example 6

A pre-treatment was performed on the model spent dialysate using electrodialysis as illustrated in Fig. 7. By the pre-treatment, the amount of electrolytes was reduced around 50%, i.e. to an osmolarity of 0.133 osmol/kg.

The pre-treated spent dialysate was used in the setup of example 5 at a temperature of 36.5°C, applying a transmembrane pressure of 3 bar and addition of sodium bicarbonate (15mg/min). The experiments showed a recovery rate of 95% as the average of 2 runs.

### Example 7

The experiment of example 6 was repeated using a simulated spent dialysate pre-treated with electrodialysis. The simulated spent dialysate was obtained by mixing 10 litre of real spent dialysate from Rigshospitalet with 10 litre of warm RO water.

An experimental setup similar to example 5 and 6 was applied using a temperature of the spent dialysate of 35°C to 42°C and a feed rate of 500 ml/min. The feed and draw pumps were turned on and the valve was adjusted to create a TMP on 3 bar. The addition of sodium bicarbonate was performed at a flow rate of 15mg/min. The experiments showed a recovery rate of 90% ± 2% as the average of 2 runs.

### Example 8

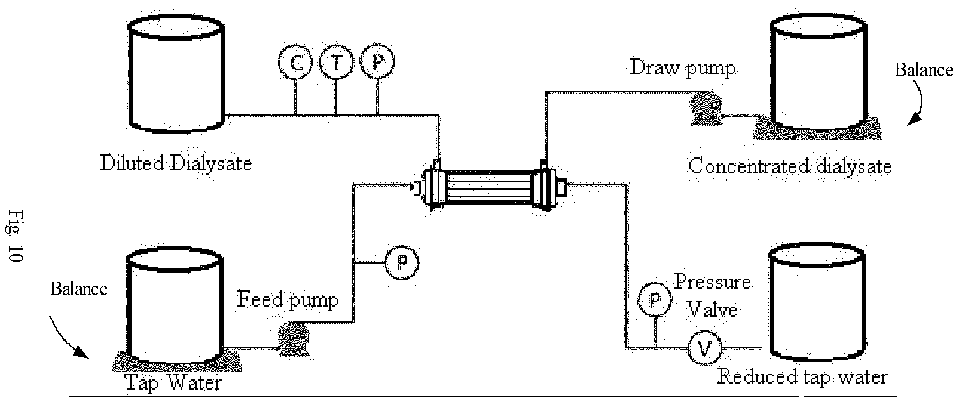

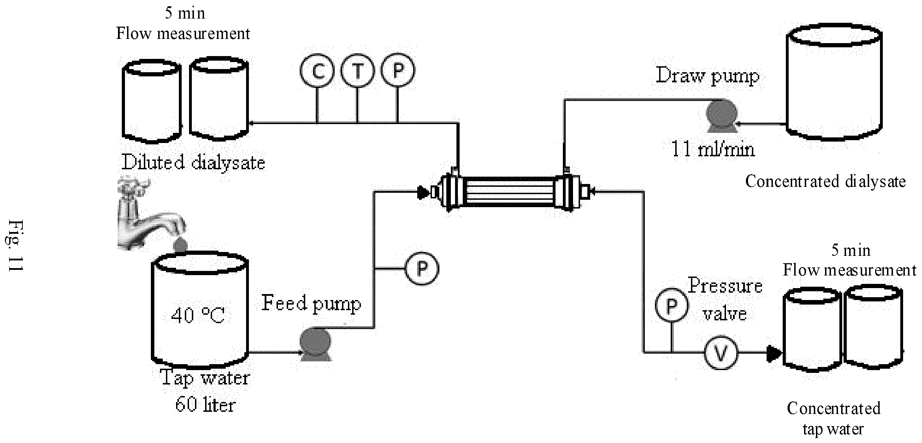

The general experimental setup for using tap water as a water source is shown in fig. 10 and Fig. 11.

Experiments on 500 ppm and 2000 ppm NaCl solutions were conducted with a recovery of 60% and 80% and a theoretical feed flow of 790 ml/min and 592.5 ml/min feed flow rate, respectively. The 500 ppm NaCl solution consists of 15 g NaCl and 30 l RO water; the 2000 ppm NaCl solution consists of 60 g NaCl in 30 l RO water.

Screening for precipitation was performed on tap water to determine how recovery, conductivity and dilution rate affects complete hemodialysis sessions.

The purity of the diluted dialysate with a dilution ratio of 1:35 and 1:45 for the first and second solutions, respectively, before mixing with the pure sodium bicarbonate concentration is checked by using a conductivity meter. Standard dialysate conductivity of the concentrated acid should be between 12-16 mS/cm at 37°C and 500 ml/min flow rate.

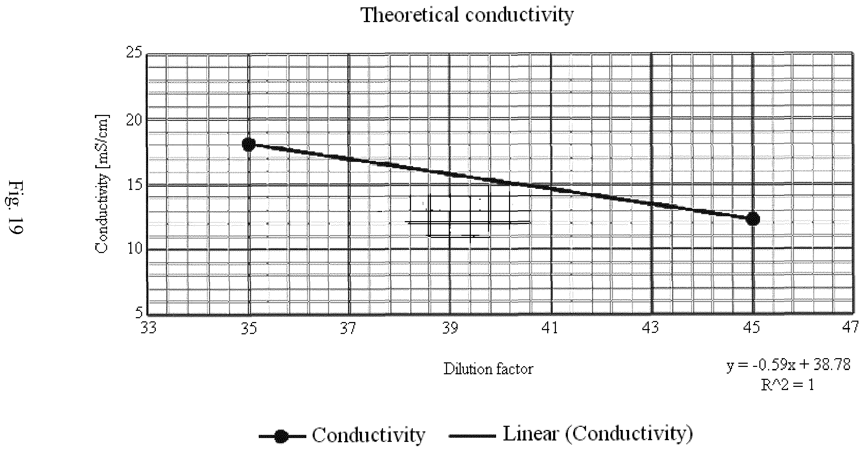

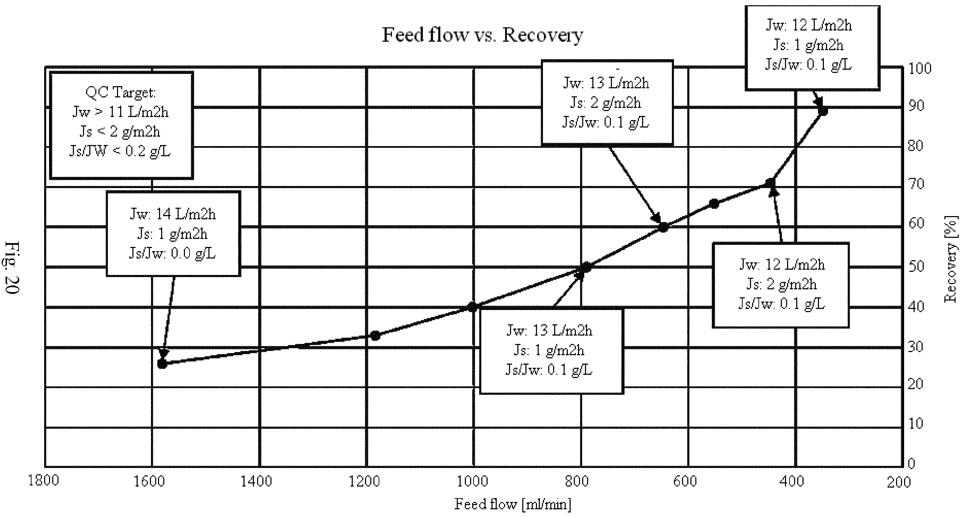

The experimental conductivity is measured (Fig. 19 and 20) and a theoretical conductivity is calculated for the two dilutions of 1:35 and 1:45 (as explained above) and RO water.

A ppm validation was performed to test the quality of tap water in Kongens Lyngby and compared with the RO water used. The theoretical ppm of spent dialysate and the pre-treated spent dialysate was found by converting the concentration used for the simulated spent dialysate solutions to ppm.

The ppm validation results as well as comparisons of the osmolarities and concentrations of different solutions used is summarized in Table 1.

**Table 1. The ppm validation as well as comparison of the osmolarities and concentrations of the different solutions used.**

| **Solution** | **Theoretical ppm** | **Experimental ppm** | **Osmolality [osm/L]** | **Concentration [g/L]** |
|---|---|---|---|---|
| Spent dialysate | 9300 | 9760 | 0.270 | 9.3 |
| Pre-treated spent dialysate | 4650 | 5030 | 0.143 | 4.65 |
| Simulated tap water | 2000 | 1940 | 0.062 | 2.0 |
| | 500 | 456 | 0.016 | 0.5 |
| Tap water DK | - | 308 | 0.010 | - |
| RO water | - | 40 | -0.002 | - |

By comparing the experimental and theoretical conductivity it is realized that there has been a negligible diffusion between the salts in the feed and draw solution.

### Example 9

Experiments on 500 ppm NaCl and 2000 ppm NaCl solutions and tap water with a recovery of 60% and 80% were conducted to identify the ideal TMP values and the feed pump RPMs for the experiments.

The ideal settings for the feed pump RMP and the TMPs are tabulated in Table 2.

**Table 2: The settings of the feed pump and the addition of TMP during each of the experiments, which has been found through screenings.**

| | **Pump flow 60% recovery** | | **Pump flow 80% recovery** | |
|---|---|---|---|---|
| **Solution** | **Feed pump RPM [min⁻¹]** | **TMP [bar]** | **Feed pump RPM [min⁻¹]** | **TMP [bar]** |
| 500 ppm | 1285 | 1 | 1235 | 1.8 |
| 2000 ppm | 1593 | 3 | 1528 | 3.9 |
| Tap water | 1675 | 3.4 | 1580 | 4 |

The data in the table shows that the permeation target and the recovery target of the 500 ppm and 2000 ppm NaCl solutions are very close to the desired permeation of 474 ml/min for the 60% recovery and the 474 ml/min for the 80% recovery.

### Example 10

An experiment was conducted to determine if the permeation of 474 ml/min and the desired dilution rate of 1:45 for tap water with up to 2000 ppm of NaCl is observed during an FO process with a target recovery of 60%.

The results are tabulated in Table 3.

**Table 3. The results of the experiments conducted on 500 ppm and 2000 ppm NaCl solutions with a 60% recovery, n=2.**

| **Solution** | **Feed in [ml/min]** | **Draw out [ml/min]** | **Permeation [ml/min]** | **Recovery [%]** | **Exp. Conduc. [mS/cm]** | **Theo. Conduc. [mS/cm]** | **Dilution rate** |
|---|---|---|---|---|---|---|---|
| 500 ppm | 792.5 *±* 0.5 | 480 *±* 1 | 468.5 *±* 1.5 | 59 *±* 0 | 13.55 *±* 0.05 | 12.23 | 1:45 |
| 2000 ppm | 784.5 *±* 1.5 | 492.5 *±* 2.5 | 481 *±* 3 | 61.5 *±* 0.5 | 13.55 *±* 0.15 | 11.56 | 1:46 |
| Target | 790 | 485 | 474 | 60 | 12-18 | 12-18 | 1:45 |

An additional experiment was conducted to determine if the permeation of 474 ml/min and the desired dilution rate of 1:45 for tap water with up to 2000 ppm of NaCl is observed during an FO process with a target recovery of 80%.

The results are tabulated in Table 4.

**Table 4. The results of the experiments conducted on 500 ppm and 2000 ppm NaCl solutions with an 80% recovery, n=2.**

| **Solution** | **Feed in [ml/min]** | **Draw out [ml/min]** | **Permeation [ml/min]** | **Recovery [%]** | **Exp. Conduc. [mS/cm]** | **Theo. Conduc. [mS/cm]** | **Dilution rate** |
|---|---|---|---|---|---|---|---|
| 500 ppm | 593.5 *±* 0.5 | 485.5 *±* 3.5 | 473.5 *±* 3.5 | 80.5 *±* 0.5 | 13.7 *±* 0.1 | 11.94 | 1:46 |
| 2000 ppm | 589.5 *±* 0.5 | 492 *±* 3 | 470 *±* 3 | 82 *±* 1 | 13.55 *±* 0.15 | 11.59 | 1:46 |
| Target | 592.5 | 485 | 474 | 80 | 12-18 | 12-18 | 1:45 |

The results for both 60% and 80% are within a reasonable range of the target value.

### Example 11

A range of recovery values were tested from 20% to 90% recovery using tap water over 5 mins of running time for each recovery values and the results are tabulated Fig. 20.

The data shows no scaling or plugging of the membrane during the screening; hence it is concluded that experiments can be run at all recoveries.

### Example 12

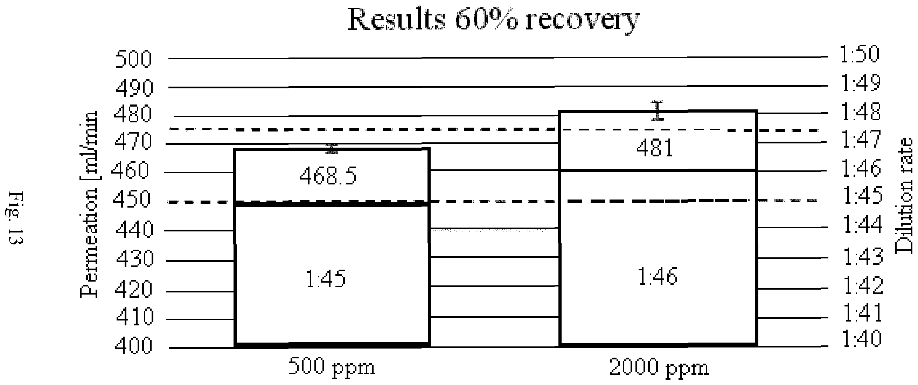

Experiments on tap water were conducted with a continuous flow of tap water through the system to determine the possibility of running a FO process for a complete hemodialysis treatment session without precipitation (Fig. 13 to Fig. 18).

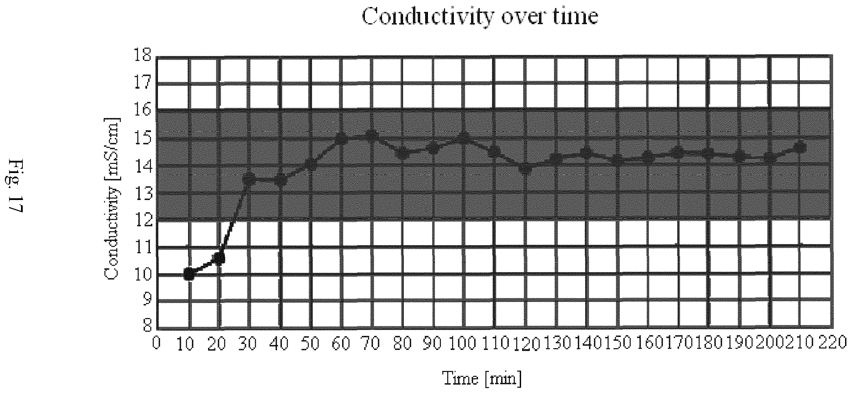

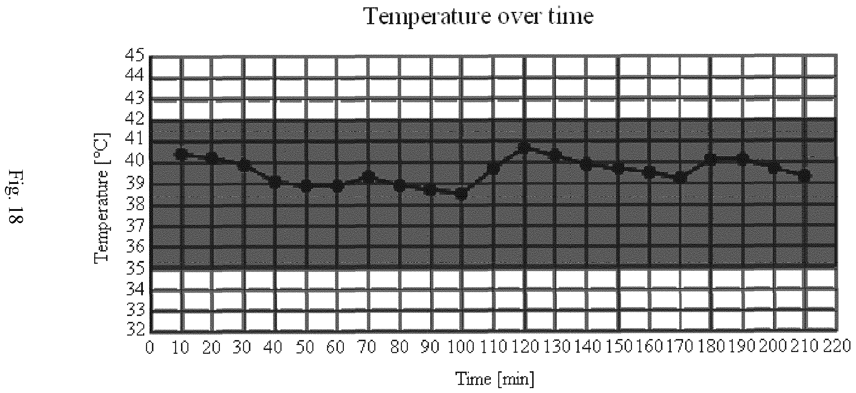

Measurements were taken in 10 mins intervals over 210 minutes of running time. The experimental results are a bit lower than the desired 60% recovery (Fig. 13), however, within the accepted the target range and proves the viability of using an FO process during the entire duration of a hemodialysis treatment. The conductivity (Fig.17) and temperature (Fig. 18) of the experiments stayed within the accepted target range throughout the duration of the experiments.

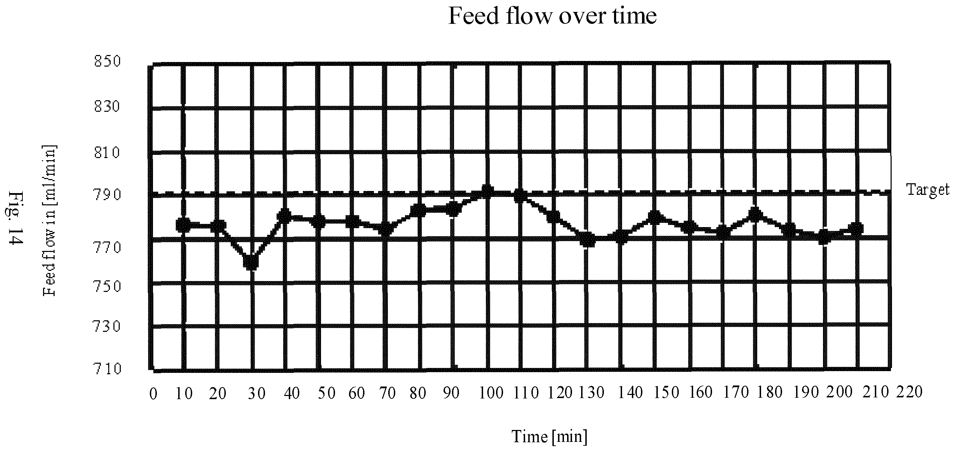

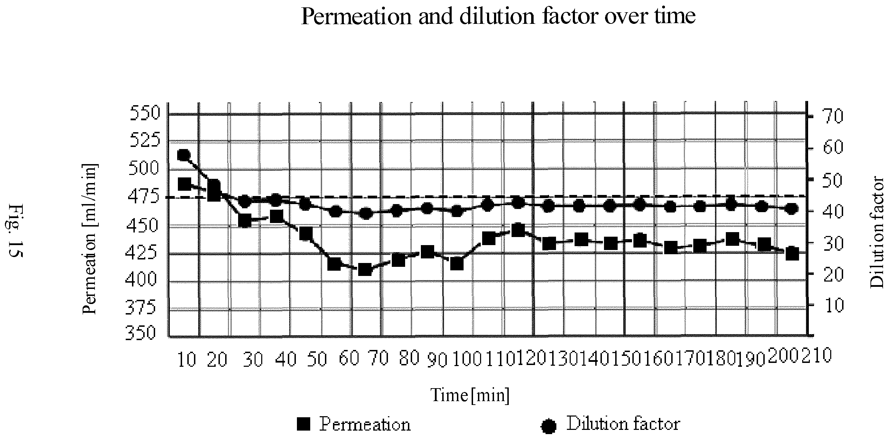

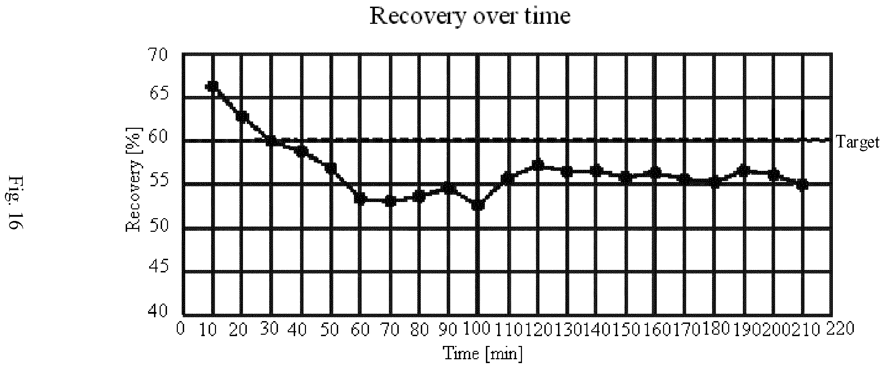

The feed flow (Fig. 14) was also measured at 3.4 bar pressure and it was concluded that the targeted feed flow is slightly lower than the target feed flow of 11 ml/min. Permeation and dilution factor study results (Fig. 15) over time show that while the permeation is not high enough to obtain a dilution ratio of 1:45, it is still within the recommended range of ratio of 1:35 to 1:45. Hence, while the permeation and recovery (Fig. 16) is lower than desired, the FO process is still viable for use for hemodialysis treatment.

## Claims

1. A method for producing a concentrated spent dialysate comprising the steps of
reducing the amount of electrolytes in a spent dialysate by electrodialysis
**characterized in that** the method further comprises the step of
de-watering the spent dialysate by a forward osmosis operation, in which the spent dialysate is passed through the feed side of a first forward osmosis unit, whereas a concentrated dialysate is passed through the draw side of the first forward osmosis unit producing a diluted concentrated dialysate, which is delivered to a hemodialysis treatment apparatus comprising a dialyzer, optionally after adjustment of the concentration of the components.

2. The method according to claim 1, wherein the amount of electrolytes in the spent dialysate is reduced by at least 20% by the electrodialysis.

3. The method according to anyone of the preceding claims, wherein the first forward osmosis unit is a first hollow fiber module, which is connected with second or further hollow fiber module(s), such that the partly de-watered spent dialysate solution exiting the feed side of the first hollow fiber module is further de-watered in the feed side of the second or further hollow fiber modules.

4. The method according to claim 3, wherein the concentrated dialysate prior to dilution in the first hollow fiber module has been diluted in second or further hollow fiber module(s).

5. The method according to claim 3 or 4, wherein the membrane area of the first membrane module is larger than the membrane area of the second or further membrane module.

6. The method according to anyone of the preceding claims, wherein the spent dialysate is treated by forward osmosis at a temperature of 30°C or above.

7. The method according to anyone of the claims 3 to 5, wherein a sodium bicarbonate solution is added to the partly diluted dialysate between the first hollow fiber module and the second or the further hollow fiber module(s), or between the second hollow fiber module and the further hollow fiber module(s).

8. The method according to anyone of the preceding claims, wherein the membrane of the forward osmosis unit comprises aquaporin water channels.

9. Hemodialysis treatment apparatus comprising
a) an ultrafiltration unit allowing for exchange of solutes of a patient's blood plasma and a dialysate, resulting in a stream of cleaned blood for returning to the patient and a stream of spent dialysate;
b) a pump for pumping blood plasma from the patient to the ultrafiltration unit,
c) an electrodialysis device capable of reducing the amount of electrolytes in the spent dialysate, **characterized by** a first forward osmosis unit comprising a membrane having a feed side and a draw side, the membrane being substantially impervious to solutes and essentially allowing only water to permeate, wherein a stream of spent dialysate from the ultrafiltration unit is in fluid communication with the feed side and a stream of concentrated dialysate is in fluid communication with the draw side, resulting in a stream of dialysate, optionally after adjustment of the concentration of the solutes, for use in the ultrafiltration unit and a stream of concentrated spent dialysate; and
wherein, optionally, the forward osmosis unit is a hollow fiber module.

10. Hemodialysis treatment apparatus according to claim 9, wherein the feed side of the first forward osmosis unit is connected to the feed side of a second or further forward osmosis units, such that, when in use, the partly de-watered spent dialysate solution exiting the first forward osmosis unit is further de-watered in the feed side of the second or further hollow fiber modules.

11. The hemodialysis treatment apparatus according claim 10, wherein the first forward osmosis unit has a membrane area twice as large as the second or further membrane module(s).

12. The hemodialysis treatment apparatus according to any of the claims 9 to 11 comprising a pump configured for providing a transmembrane pressure in the forward osmosis unit of 1 bar or more.

13. The hemodialysis treatment apparatus according to any one of claims 9 to 12, wherein a conduit between the first forward osmosis and the second or further osmosis unit(s) is supplemented with a further conduit, for connecting to a source of sodium bicarbonate.

14. The hemodialysis treatment apparatus according to any of the claims 9 to 13, further comprising a pump for pumping the concentrated dialysate to the forward osmosis unit, or a pump for suction of the diluted dialysate from the forward osmosis unit.

15. The hemodialysis treatment apparatus according to anyone of the claims 9 to 14, further comprising:
a mixing device for adjusting the dialysate from the forward osmosis unit with a sodium carbonate solution and, optionally,
a conductivity sensor after the mixing device for feed-back regulation of the adjustment of the dialysate from the forward osmosis unit with the sodium carbonate solution.

## Patentansprüche

1. Verfahren für die Herstellung eines konzentrierten verbrauchten Dialysats, umfassend die Schritte des
Reduzierens der Menge von Elektrolyten in einem verbrauchten Dialysat durch Elektrodialyse,
**dadurch gekennzeichnet, dass** das Verfahren ferner die Schritte umfasst des
Entwässerns des verbrauchten Dialysats durch einen Vorwärtsosmosearbeitsvorgang, wobei das verbrauchte Dialysat durch die Zufuhrseite einer ersten Vorwärtsosmoseeinheit geführt wird, während ein konzentriertes Dialysat durch die Zugseite der ersten Vorwärtsosmoseeinheit geführt wird, wodurch ein verdünntes konzentriertes Dialysat erzeugt wird, das zu einem Hämodialysebehandlungsapparat, der einen Dialysator umfasst, wahlweise nach Einstellen der Konzentration der Komponenten geliefert wird.

2. Verfahren nach Anspruch 1, wobei die Menge von Elektrolyten in dem verbrauchten Dialysat durch die Elektrodialyse um mindestens 20 % reduziert wird.

3. Verfahren nach einem der vorhergehenden Ansprüche, wobei die erste Vorwärtsosmoseeinheit ein erstes Hohlfasermodul ist, das mit einem zweiten oder weiteren Hohlfasermodul(en) derart verbunden ist, dass die teilweise entwässerte verbrauchte Dialysatlösung, die aus der Zufuhrseite des ersten Hohlfasermoduls austritt, noch weiter in der Zufuhrseite des zweiten oder von weiteren Hohlfasermodulen entwässert wird.

4. Verfahren nach Anspruch 3, wobei das konzentrierte Dialysat, vor der Verdünnung in dem ersten Hohlfasermodul, in dem zweiten oder dem/den weiteren Hohlfasermodul(en) verdünnt worden ist.

5. Verfahren nach Anspruch 3 oder 4, wobei der Membranbereich des ersten Membranmoduls größer ist als der Membranbereich des zweiten oder weiteren Membranmoduls.

6. Verfahren nach einem der vorhergehenden Ansprüche, wobei das verbrauchte Dialysat wodurch Vorwärtsosmose bei einer Temperatur von 30 °C oder darüber behandelt wird.

7. Verfahren nach einem der Ansprüche 3 bis 5, wobei eine Natriumbicarbonatlösung dem teilweise verdünnten Dialysat zwischen dem ersten Hohlfasermodul und dem zweiten oder dem/den weiteren Hohlfasermodul(en) oder zwischen dem zweiten Hohlfasermodul und dem/den weiteren Hohlfasermodul(en) zugegeben wird.

8. Verfahren nach einem der vorhergehenden Ansprüche, wobei die Membran der Vorwärtsosmoseeinheit Aquaporinwasserkanäle umfasst.

9. Hämodialysebehandlungsapparat umfassend
a) eine Ultrafiltriereinheit, die den Austausch von gelösten Substanzen eines Patientenblutplasmas und eines Dialysats gestattet, was zu einem Strom von gereinigtem Blut zum Rückführen zu dem Patienten und einem Strom von verbrauchtem Dialysat führt;
b) eine Pumpe zum Pumpen von Blutplasma von dem Patienten zu der Ultrafiltriereinheit
c) eine Elektrodialysevorrichtung, die in der Lage ist, die Menge von Elektrolyten in dem verbrauchten Dialysat zu reduzieren, **gekennzeichnet durch** eine erste Vorwärtsosmoseeinheit, die eine Membran umfasst, die eine Zufuhrseite und eine Zugseite aufweist, wobei die Membran im wesentlichen für gelöste Substanzen undurchlässig ist und im wesentlichen nur Wasser gestattet, hindurchzugehen, wobei ein Strom von verbrauchtem Dialysat aus der Ultrafiltriereinheit sich in Fluidkommunikation mit der Zufuhrseite befindet und ein Strom von konzentriertem Dialysat sich in Fluidkommunikation mit der Zugseite befindet, was, wahlweise nach Einstellen der Konzentration der der gelösten Substanzen, zu einem Strom von Dialysat zur Verwendung in der Ultrafiltriereinheit und einem Strom von konzentriertem verbrauchtem Dialysat führt; und
wobei wahlweise die Vorwärtsosmoseeinheit ein Hohlfasermodul ist.

10. Hämodialysebehandlungsapparat nach Anspruch 9, bei die Zufuhrseite der ersten Vorwärtsosmoseeinheit mit der Zufuhrseite einer zweiten oder weiteren Vorwärtsosmoseeinheiten derart verbunden ist, dass, bei der Verwendung, die teilweise entwässerte verbrauchte Dialysatlösung, die aus der ersten Vorwärtsosmoseeinheit austritt, in der Zufuhrseite der zweiten oder weiterer Hohlfasermodule noch weiter entwässert wird.

11. Hämodialysebehandlungsapparat nach Anspruch 10. wobei die erste Vorwärtsosmoseeinheit einen Membranbereich aufweist, der zweimal so groß wie das zweite oder weitere Membranmodul(e) ist.

12. Hämodialysebehandlungsapparat nach einem der Ansprüche 9 bis 11, eine Pumpe umfassend, die zum Bereitstellen eines Transmembrandrucks in der Vorwärtsosmoseeinheit von 1 bar oder mehr konfiguriert ist.

13. Hämodialysebehandlungsapparat nach einem der Ansprüche 9 bis 12, wobei die Leitung zwischen der ersten Vorwärtsosmose- und der/den zweiten oder weiteren Osmoseeinheit(en) mit einer weiteren Leitung zum Verbinden mit einer Quelle von Natriumbicarbonat ergänzt ist.

14. Hämodialysebehandlungsapparat nach einem der Ansprüche 9 bis 13, ferner eine Pumpe zum Pumpen des konzentrierten Dialysats zu der Vorwärtsosmoseeinheit oder eine Pumpe zum Saugen des verdünnten Dialysats von der Vorwärtsosmoseeinheit umfassend.

15. Hämodialysebehandlungsapparat nach einem der Ansprüche 9 bis 14, ferner Folgendes umfassend:
eine Mischvorrichtung zum Einstellen des Dialysats von der Vorwärtsosmoseeinheit mit einer Natriumcarbonatlösung und, wahlweise,
einen Leitfähigkeitssensor nach der Mischvorrichtung zur Rückmeldungsregulierung der Einstellung des Dialysats von der Vorwärtsosmoseeinheit mit der Natriumcarbonatlösung.

## Revendications

1. Procédé de production d'un dialysat usé concentré comprenant les étapes de
réduction de la quantité d'électrolytes dans un dialysat usé par électrodialyse
**caractérisé en ce que** le procédé comprend en outre l'étape de
déshydratation du dialysat usé par une opération d'osmose directe, dans laquelle le dialysat usé est passé à travers le côté d'alimentation d'une première unité d'osmose directe, alors qu'un dialysat concentré est passé à travers le côté de soutirage de la première unité d'osmose directe produisant un dialysat concentré dilué, qui est délivré à un appareil de traitement d'hémodialyse comprenant un dialyseur, éventuellement après ajustement de la concentration des composants.

2. Procédé selon la revendication 1, dans lequel la quantité d'électrolytes dans le dialysat usé est réduit d'au moins 20 % par l'électrodialyse.

3. Procédé selon l'une quelconque de revendications précédentes, dans lequel la première unité d'osmose directe est un premier module à fibre creuse, qui est connecté avec un deuxième ou un(des) autre(s) module(s) à fibre creuse, de telle manière que la solution de dialysat usé partiellement déshydraté sortant du côté d'alimentation du premier module à fibre creuse est en outre déshydraté dans le côté d'alimentation du deuxième ou autres modules à fibre creuse.

4. Procédé selon la revendication 3, dans lequel le dialysat concentré avant la dilution dans le premier module à fibre creuse a été dilué dans le deuxième ou autre(s) module(s) à fibre creuse.

5. Procédé selon la revendication 3 ou 4, dans lequel la surface de membrane du premier module à membrane est plus grande que la surface de membrane du deuxième ou autre module à membrane.

6. Procédé selon l'une quelconque des revendications précédentes, dans lequel le dialysat usé est traité par osmose directe à une température de 30 °C ou au-dessus.

7. Procédé selon l'une quelconque des revendications 3 à 5, dans lequel une solution de bicarbonate de sodium est ajoutée au dialysat partiellement dilué entre le premier module à fibre creuse et le deuxième ou autre(s) module(s) à fibre creuse ou entre le deuxième module à fibre creuse et l' (les) autre(s) module(s) à fibre creuse.

8. Procédé selon l'une quelconque des revendications précédentes, dans lequel la membrane de l'unité d'osmose directe comprend des canaux à eau d'aquaporine.

9. Appareil de traitement d'hémodialyse comprenant
a) une unité d'ultrafiltration permettant l'échange de solutés du plasma du sang d'un patient et d'un dialysat, résultant dans un courant de sang nettoyé pour le retour au patient et un courant de dialysat usé ;
b) une pompe pour pomper le plasma du sang du patient à l'unité d'ultrafiltration,
c) un dispositif d'électrodialyse capable de réduire la quantité d'électrolytes dans le dialysat usé, **caractérisé par** une première unité d'osmose directe comprenant une membrane ayant un côté d'alimentation et un côté de soutirage, la membrane étant essentiellement imperméable aux solutés et permettant essentiellement seulement à l'eau d'être perméable, un courant du dialysat usé de l'unité d'ultrafiltration étant en communication fluide avec le côté d'alimentation et un courant de dialysat concentré étant en communication fluide avec le côté de soutirage, résultant dans un courant de dialysat, éventuellement après ajustement de la concentration des solutés, pour une utilisation dans l'unité d'ultrafiltration et un courant de dialysat usé concentré ; et
éventuellement, l'unité d'osmose directe étant un module à fibre creuse.

10. Appareil de traitement d'hémodialyse selon la revendication 9, dans lequel le côté d'alimentation de la première unité d'osmose directe est connecté au côté d'alimentation d'une deuxième ou autres unités d'osmose directe, de telle manière que, quand elle est utilisée, la solution de dialysat usé partiellement déshydraté sortant de la première unité d'osmose directe est en outre déshydratée dans le côté d'alimentation du deuxième ou autres modules à fibre creuse.

11. Appareil de traitement d'hémodialyse selon la revendication 10, dans lequel la première unité d'osmose directe a une surface de membrane deux fois aussi grande que le deuxième ou autre (s) module(s) à membrane.

12. Appareil de traitement d'hémodialyse selon l'une quelconque des revendications 9 à 11 comprenant une pompe configurée pour fournir une pression transmembranaire dans l'unité d'osmose directe de 1 bar ou plus.

13. Appareil de traitement d'hémodialyse selon l'une quelconque des revendications 9 à 12, dans lequel un conduit ente la première unité d'osmose directe et la deuxième ou autre(s) unité(s) d'osmose est complémentée avec un autre conduit, pour la connexion à une source de bicarbonate de sodium.

14. Appareil de traitement d'hémodialyse selon l'une quelconque des revendications 9 à 13, comprenant en outre une pompe pour pomper le dialysat concentré à l'unité d'osmose directe ou une pompe pour la succion du dialysat dilué de l'unité d'osmose directe.

15. Appareil de traitement d'hémodialyse selon l'une quelconque des revendications 9 à 14, comprenant en outre :
un dispositif de mélange pour ajuster le dialysat de l'unité d'osmose directe avec une solution de bicarbonate de sodium et, éventuellement,
un capteur de conductivité après le dispositif de mélange pour la retroregulation de l'ajustement du dialysat de l'unité d'osmose directe avec la solution de bicarbonate de sodium.
